# EUROPEAN PATENT SPECIFICATION

(11) **EP 3 402 333 B1**
(45) Date of publication and mention of the grant of the patent: **30.10.2019**
(21) Application number: 17701804.1
(22) Date of filing: 12.01.2017
(51) Int. Cl.: A01N 43/08, A01N 43/36, A01N 43/40, A01N 43/60, A01N 43/84, A01N 43/90, A01P 15/00

(54) **USE OF ACTIVE SUBSTANCES FOR CONTROLLING VIRUS INFECTION IN PLANTS**
VERWENDUNG VON WIRKSUBSTANZEN ZUR STEUERUNG VON VIRUSINFEKTION BEI PFLANZEN
UTILISATION DE SUBSTANCES ACTIVES POUR CONTRÔLER UNE INFECTION PAR UN VIRUS DANS DES PLANTES

(30) Priority: 13.01.2016 EP 16290008
(43) Date of publication of application: 21.11.2018
(73) Proprietor: Bayer CropScience Aktiengesellschaft, 40789 Monheim am Rhein (DE)
(72) Inventor: BALTZ, Rachel, 69660 Collonges au Mont d'Or (FR); BERNIER, David, 69004 Lyon (FR); JAY-BRIOUDES, Florence, 8044 Zürich (CH); KNOBLOCH, Thomas, 69380 Alix (FR); VITEL, Maxime, 69270 Fontaines sur Saône (FR); VOINNET, Olivier, 8057 Zürich (CH)
(74) Representative: Bosser, Isabelle
(86) International application number: PCT/EP2017/050583
(87) International publication number: WO 2017/121809

(56) References cited:
- EP-A1- 2 902 387
- CN-A- 102 746 282
- I. S. FEDOROVICH ET AL: "Thioamides from 5-arylfurfural and monosubstituted piperazine derivatives (Wilgerodt-Kindler reaction)", RUSSIAN JOURNAL OF ORGANIC CHEMISTRY., vol. 43, no. 8, 1 August 2007 (2007-08-01) , pages 1190-1195, XP055260243, US ISSN: 1070-4280, DOI: 10.1134/S1070428007080180
- Adnan Younis ET AL: "RNA Interference (RNAi) Induced Gene Silencing: A Promising Approach of Hi-Tech Plant Breeding", International Journal of Biological Sciences, vol. 10, no. 10, 1 January 2014 (2014-01-01), pages 1150-1158, XP055273136, Australia ISSN: 1449-2288, DOI: 10.7150/ijbs.10452

## Description

### TECHNICAL FIELD

The present invention relates to the use of active substances for stimulating the natural defense mechanism of plants against viruses in order to control virus infections in plants and to methods for controlling virus infections in plants. The present invention also pertains to the use of active substances for activating the RNA-interference-based natural defense mechanism (also designated herein as RNA-silencing-based natural defense mechanism) of plants against viruses.

### BACKGROUND

Plant viruses are responsible for major crop damages around the world. Indeed, some virus families, such as the Potyviridae, cause critical yield losses both in developed and emerging countries, conflicting with the ever-increasing food demand. To limit virus infections and spread, disease management is mainly conducted via prevention, as curative treatments are not or poorly efficient.

Several compounds have been identified for trying to control plant viruses. WO2011/030816 teaches the use of certain ascorbic acid derivatives to control certain plan viruses. WO2012/016048 provides for the use of azide-modified biomolecules as antiviral agents, including against plant viruses. WO2014/050894 teaches the use of other ascorbic acid related compounds to control plant viruses.

Plants have evolved to continuously cope with threats using their available resources and balancing them between growth or defense against biotic and abiotic threats. RNA silencing plays a major part in this balance by dynamically linking developmental programs and environmental stress responses to gene expression changes through transcriptional gene silencing (TGS) and post-transcriptional gene silencing (PTGS). Disease resistance in plants relies on preformed barriers, toxic secondary metabolites and inducible defense mechanisms. Upon pathogen recognition, plants often initiate hypersensitive response, leading to cell death at the infection site and preventing the pathogen from spreading. In addition, pathogen detection triggers various inducible systemic defenses, in parts of the plant distant from the primary infection site. This process, known as Systemic Acquired Resistance (SAR), is effective in many plant species. The resistance achieved is long-lasting and effective against subsequent infections by a broad range of pathogens e.g. fungi, bacteria and viruses.

The Strobilurin class of fungicides comprises a variety of synthetic plant-protecting compounds with broad-spectrum. In 2002, the strobilurin Pyraclostrobin has been demonstrated to enhance the resistance of tobacco against infection by either tobacco mosaic virus (TMV) or the wildfire pathogen *Pseudomonas syringae pv tabaci* (Herms et al., Plant Physiology 2002, 130: 120-127). Pyraclostrobin was also able to enhance TMV resistance in NahG transgenic tobacco plants unable to accumulate significant amounts of the endogenous salicylic acid. Pyraclostrobin enhances TMV resistance in tobacco either by acting downstream of Salicylic Acid (SA) in the SA signaling mechanism or by functioning independently of SA. The latter assumption is the more likely because, in infiltrated leaves, Pyraclostrobin did not cause the accumulation of SA-inducible pathogenesis-related (PR)-1 proteins that often are used as conventional molecular markers for SA-induced disease resistance. Application of strobilurins is described either alone (WO 01/82701) or in mixture with metiram (WO 2007/104669). Among the plant defense responses to phytoviruses, the antiviral RNA silencing pathway is the broadest defense system affecting both the local and the systemic accumulation of a wide range of viruses. RNA silencing is a mechanism that directly defends plant host cells against exogenous nucleic acids, including viruses and transposable elements. This defense is triggered by double-stranded RNA (dsRNA), derived from amplification of invasive nucleic acids, which is processed by the host into small interfering RNAs (siRNAs) that are 20-24 nucleotides (nt) in size. These siRNAs are then used to guide the silencing of the viral or transposable element RNA or DNA through PTGS or TGS, respectively.

RNA silencing is then a potent antiviral mechanism whereby small interfering siRNAs processed by the enzyme Dicer from viral double-stranded RNA replication intermediates are loaded into ARGONAUTE effector proteins and turned back onto the invader's RNA genome to induce its degradation. This innate immune response is remarkably versatile because, being solely programmed by structural and nucleotide-sequence genomic features, it can respond to virtually any plant virus (Shimura et al., 2011, Biochimica et Biophysica Acta 1809: 601-612). Attesting the importance of RNA silencing in plant defense, plants impaired in siRNA production or activity are hyper-susceptible to phytoviruses, and conversely many viruses have evolved suppressors of RNAi in order to maintain virulence (Voinnet O. et al, Nature Review Microbiology 2013 Nov;11(11):745-60).

Although certain compounds have been identified in the past as potential inducers of certain plant defense mechanisms against viruses, there remains a need to provide active substances suitable for stimulating the natural defense mechanism of plants against viruses, in particular for stimulating the broad non-specific RNA-silencing-based defense mechanism of plants against viruses in order to control viral diseases in plants.

### FIGURES

Figure 1 illustrates the qPCR analysis of the SUL mRNA relative levels in SUC-SUL plants seven days after treatment with DMSO only or DFPM, normalized to EXP10. Error bars represent standard deviation from three independent experiments.
Figure 2 represents the average number of infection foci at five days post-infection on inoculated leaves. Error bars represent standard deviation from twelve plants.
Figure 3 illustrates the RT-qPCR analyses on HcPro mRNA accumulation in systemic tissues at six days post-infection. Error bars represent standard deviation from three independent PCR results.

### DEFINITIONS

The term "(C₁-C₆)alkyl" as used herein refers to a branched- or straight-chain alkyl group containing from 1 to 6 carbon atoms. Representative examples of (C₁-C₆)alkyl include methyl, ethyl, n-propyl, i-propyl and the different butyl, pentyl or hexyl isomers.

The term "halogen" as used herein refers to a fluorine, chlorine, iodine or bromine atom.

The term "(C₁-C₆)haloalkyl" as used herein refers to a (C₁-C₆)alkyl group, as defined above, wherein at least one hydrogen atom is replaced with a halogen atom. Representative examples of (C₁-C₆)haloalkyl include -CF₃,- CH₂Cl and -CH₂-CF₃.

The term "(C₁-C₆)cyanoalkyl" as used herein refers to a (C₁-C₆)alkyl group, as defined above, wherein at least one hydrogen atom is replaced with a cyano group.

The term "(C₃-C₆)cycloalkyl" as used herein refers to a saturated monocyclic alkyl radical wherein the cyclic framework has 3 to 6 carbon atoms. Representative examples of "(C₃-C₆)cycloalkyl" include cyclopropyl, cyclobutyl and cyclopentyl.

The term "azabicycloalkyl" as used herein refers to a fully saturated monovalent radical containing from 5 and up to 10 carbon atoms and a nitrogen atom in a bicyclic ring. Representative exemples of "azabicycloalkyl" include azabicyclo[3.1.0]hexyl, azabicyclo[2.2.1]heptyl and azabicyclo[2.2.2]octyl.

The expression "may be substituted" as used herein (e.g. a phenyl that may be substituted), means that the substitution is optional and therefore includes both unsubstituted and substituted atoms or groups (e.g. unsubstituted and substituted phenyl). When an atom or group is substituted, any hydrogen on the designated atom or group can be replaced with a substituent (up to and including that every hydrogen is replaced with a substituent) provided that the normal valency of the designated atom or group is not exceeded and that the substitution results in a stable compound.

The term "active substance" as used herein designates a compound of formula (I) as described herein or any mixtures thereof.

### DETAILED DESCRIPTION

It has now been found that compounds of formula (I) as disclosed herein are suitable for stimulating the natural defense mechanism of plants against viruses, in particular for stimulating the RNA-silencing-based defense mechanism of plants against viruses. Therefore, the compounds of formula (I) as disclosed herein may be useful for controlling viral diseases in plants. The term "control" or "controlling" as used herein designates a preventive or curative control.

Accordingly, the present invention relates to a method for controlling viral diseases in plants, more specifically to a method for stimulating the natural defense mechanism of plants against viruses, in particular for stimulating the RNA-silencing-based defense mechanism of plants against viruses. The method comprises applying to the plants one or more compounds of formula (I): 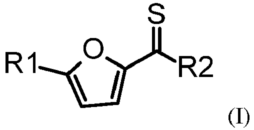 wherein
- R1 is a phenyl that may be substituted with one or more halogens, preferably chlorine and bromine;
- R2 is a -NR'R" group wherein :
   - R' and R" are independently selected from the group consisting of (C₁-C₆)alkyl (preferably methyl, ethyl and n-propyl) and (C₃-C₆)cycloalkyl (preferably c-pentyl), or
   - R' and R" together with the nitrogen atom to which they are attached form a 5- or 6-membered heterocycle chosen from pyrrolidine, piperidine, morpholine, or piperazine, wherein said heterocycle may be substituted with one or more substituents selected from the group consisting of halogen, (C₁-C₆)alkyl and (C₁-C₆)cyanoalkyl, or
   - R' and R" together with the nitrogen atom to which they are attached form an azabicycloalkyl that may be substituted with a (C₁-C₆)haloalkyl, preferably an azabicyclo[3.1.0]hexyl that may be substituted with a (C₁-C₆)haloalkyl.

In some embodiments, the active substance is a compound of formula (I) wherein :
- R1 is a phenyl substituted with one or two halogens independently chosen from chlorine or bromine ; and/or
- R2 is a -NR'R" group wherein :
   - R' and R" are independently selected from the group consisting of methyl, ethyl, n-propyl and cyclopentyl, or
   - R' and R" together with the nitrogen atom to which they are attached form a 5- or 6-membered heterocycle chosen from pyrrolidine, piperidine, morpholine or piperazine, wherein said heterocycle may be substituted with one or more substituents selected from the group consisting of methyl, fluorine and cyanoethyl, or
   - R' and R" together with the nitrogen atom to which they are attached form an azabicyclo[3.1.0]hexyl substituted with a (C₁-C₆)haloalkyl, such as a trifluoromethyl.

In some preferred embodiments, the active substance is a compound of formula (I) wherein :
- R1 is selected from the group consisting of 3-chlorophenyl, 4-chlorophenyl, 4-bromophenyl, 2,3-dichlorophenyl and 3,4-dichlorophenyl ; and/or
- R2 is selected from the group consisting of -NEt₂, -N(n-Pr)₂, -N(c-pentyl)Me, piperidin-1-yl, pyrrolidin-1-yl, N-morpholino, 4,4-difluoropiperidin-1-yl, , 4-(2-cyanoethyl)-piperazin-1-yl, 2,6-dimethylmorpholin-4-yl and 6-(trifluoromethyl)-3-azabicyclo[3.1.0]hex-3-yl.

In some embodiments, the active substance is a compound of formula (I) wherein R1 and R2 are selected such as to provide the specific compounds disclosed herein below in table 1.

In some preferred embodiments, the active substance is a compound of formula (I) wherein :
- R1 is 3,4-dichlorophenyl and R2 is 4,4-difluoropiperidinyl-1-yl ; or
- R1 is 3,4-dichlorophenyl and R2 is piperidin-1-yl ; or
- R1 is 4-bromophenyl and R2 is piperidin-1-yl.

Compound of formula (I) wherein R1 is 3,4-dichlorophenyl and R2 is piperidin-1-yl is known as DFPM [5-(3,4-Dichlorophenyl)Furan-2-yl]-Piperidin-1-ylMethanethione.

DFPM rapidly downregulates ABA-dependent gene expression in plant and also inhibits ABA-induced stomatal closure. DFPM also stimulates expression of plant defense-related genes including PHYTOALEXIN DEFICIENT4 (PAD4)- and ENHANCED DISEASE SUSCEPTIBILITY1 (EDS1) (Kim et al., Current Biology 2011, 21, 11:990-997).

5-Arylfuran-2-thioamides of formula (I) may be suitably prepared :
- either from 5-arylfuran-2-carboxaldehydes and amines by a Wilgerodt-Kindler reaction, as disclosed in Fedorovich, Ganushchak, Karpyak, Obushchak, Lesyuk, Russian Journal of Organic Chemistry 2007, 43(8), 1190-1195 [10.1134/S1070428007080180]
- or via thionation (using classical methods such as treatment with phosphorus pentasulfide or Lawesson reagent, see e.g. "Thionation reactions of Lawesson's reagents" : Cava, Levinson, Tetrahedron 1985, 41(22), 5061-5087 [10.1016/S0040-4020(01)96753-5]) of the corresponding 5-arylfuran-2-carboxamides, which in turn are readily obtained by literature-known methods (see e.g. Drizin, Gregg, et al., Bioorganic & Medicinal Chemistry 2008, 16(12), 6379-6386 [10.1016/j.bmc.2008.05.003] ; Gargano, Perspicace, et al., European Journal of Medicinal Chemistry 2014, 87, 203-219 [10.1016/j.ejmech.2014.09.061]).

An effective amount of active substance is typically applied to the plants. The effective amount of active substance which is applied to the plants will depend on various factors, such as the nature of the active substance, the formulation, the plants being targeted (plants nature and plants part), the application method, the purpose of the treatment (prophylactic or therapeutic) and the virus being targeted. The amount applied to the plants may suitably range from 0.01 to 5 kg/ha, or from 0.1 to 3 kg/ha, or from 0.5 to 2 kg/ha.

As indicated above, the active substance is applied to the plants. The term "plants" as used herein include plants and parts thereof, such as the aerial and/or subterraneous parts of the plants as well as the harvested material. Subterraneous plants parts include root, rhizomes, tubers, suckers, slips, seeds and seed. The aerial plant parts include stem, bark, shoot, leaf, flower, fruits, fruiting bodies, stalk, needles and branches. Thus, the active substance may be efficiently applied to the root, rhizomes, tubers, suckers, slips, seeds, seed, stem, bark, shoot, leaf, flower, fruits, fruiting bodies, stalk, needles, branches, harvested material of the plants. In alternative embodiments, the method for controlling viral diseases in plants comprises applying the disclosed active substance to the plants' habitat and/or store.

The active substance can be efficiently applied to a large variety of plants. It may be applied to plants of the varieties which are commercially available or in use. However, plant varieties are also understood as meaning plants with novel traits which have been bred either by traditional breeding, by mutagenesis or with the aid of recombinant DNA techniques and/or to plants which can be obtained by traditional breeding and optimization methods or else by biotechnological and genetic engineering methods or by combinations of these methods; this includes the transgenic plants and the plants which are capable or not of being protected by Plant Breeders' Rights.

The active substance may also be efficiently applied to genetically modified organisms (GMOs). Genetically modified plants are plants in which a heterologous gene has been stably integrated into the genome. The expression "heterologous gene" essentially means a gene which has been provided or assembled outside the plants and, when introduced into the nuclear, chloroplastic or mitochondrial genome, imparts novel or improved agronomic or other properties to the transformed plant by expressing a protein or polypeptide of interest or by down regulating or silencing another gene which is present in the plant, or other genes which are present in the plant (using, for example, antisense technology, cosuppression technology or RNAi technology [RNA interference]). A heterologous gene that is located in the genome is also called a transgene. A transgene that is defined by its particular location in the plant genome is referred to as a transformation event, or transgenic event.

All plants which have genetic material which imparts, to these plants, especially advantageous, useful traits (whether obtained by breeding and/or by biotechnology) may be treated by the disclosed method.

Plants and plant varieties which can likewise be treated in accordance with the invention are those plants which are resistant to one or more abiotic stress factors. Abiotic stress conditions can include, for example, drought, chill and heat conditions, osmotic stress, water-logging, elevated soil salt content, elevated exposure to minerals, ozone conditions, high-light conditions, limited availability of nitrogen nutrients, limited availability of phosphorus nutrients or the avoidance of shade.

Plants and plant varieties which can likewise be treated in accordance with the invention are those plants which are characterized by enhanced yield characteristics. Enhanced yield in these palm plants can be the result of, for example, improved plant physiology, improved plant growth and improved plant development, such as water utilization efficiency, water retention efficiency, improved nitrogen utilization, improved carbon assimilation, improved photosynthesis, increased germination efficiency and modified maturation. The yield can furthermore be influenced by improved plant architecture (under stress conditions and under nonstress conditions), among which early flowering, flowering control for the production of hybrid seed, seedling vigour, plant size, internode number and distance, root growth, seed size, fruit size, pod size, pod or ear number, number of seeds per pod or ear, seed mass, enhanced seed filling, reduced seed dispersal, reduced pod dehiscence, and lodging resistance. Further yield traits include seed composition, such as carbohydrate content, protein content, oil content and oil composition, nutritional value, reduction in antinutritional compounds, improved processability and improved storability.

Plants which can likewise be treated in accordance with the invention are hybrid plants that already express the characteristics of heterosis, or hybrid vigour, which generally results in higher yield, higher vigour, better health and better resistance to biotic and abiotic stress factors. Such plants are typically generated by crossing an inbred male-sterile parent line (the female parent) with another inbred male-fertile parent line (the male parent). The hybrid seed is typically harvested from the male-sterile plants and sold to growers. Male-sterile plants can sometimes (for example in maize) be generated by detasseling (i.e. the mechanical removal of the male reproductive organs or the male flowers); however, more typically, male sterility is the result of genetic determinants in the plant genome. In this case, in particular when seed is the desired product which is to be harvested from the hybrid plants, it is typically useful to ensure that male fertility in hybrid plants which contain the genetic determinants responsible for male sterility is fully restored. This can be achieved by ensuring that the male parents have appropriate fertility restorer genes which are capable of restoring male fertility in hybrid plants which contain the genetic determinants responsible for male sterility. Genetic determinants for male sterility can be located in the cytoplasm. Examples of cytoplasmic male sterility (CMS) have been described for example for Brassica species (WO 1992/005251, WO 1995/009910, WO 1998/27806, WO 2005/002324, WO 2006/021972 and US 6,229,072). However, genetic determinants for male sterility may also be located in the nuclear genome. Male-sterile plants can also be obtained by plant biotechnology methods, such as genetic engineering. A particularly advantageous means for generating male-sterile plants is described in WO 89/10396, in which, for example, a ribonuclease such as a barnase is selectively expressed in the tapetum cells in the stamens. The fertility can then be restored by expression in the tapetum cells of a ribonuclease inhibitor such as barstar (for example WO 1991/002069).

The active substance is particularly suitable for controlling viral diseases in the following plants: cotton, flax, grapevine, fruit, vegetables, such as *Rosaceae sp.* (for example pome fruits such as apples and pears, but also stone fruits such as apricots, cherries, almonds and peaches, and soft fruits such as strawberries), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (for example banana trees and plantations), *Rubiaceae sp.* (for example coffee), *Theaceae sp., Sterculiceae sp., Rutaceae sp.* (for example lemons, oranges and grapefruit); *Solanaceae sp.* (for example tomatoes), *Liliaceae sp., Asteraceae sp.* (for example lettuce), *Umbelliferae sp., Cruciferae sp., Chenopodiaceae sp., Cucurbitaceae sp.* (for example cucumber), *Alliaceae sp.* (for example leek, onion), *Papilionaceae sp.* (for example peas); major crop plants, such as *Gramineae sp.* (for example maize, turf, cereals such as wheat, rye, rice, barley, oats, millet and triticale), *Asteraceae sp.* (for example sunflower), *Brassicaceae sp.* (for example white cabbage, red cabbage, broccoli, cauliflower, Brussels sprouts, pak choi, kohlrabi, radishes, and oilseed rape, mustard, horseradish and cress), *Fabacae sp.* (for example bean, peanuts), *Papilionaceae sp.* (for example soya bean), *Solanaceae sp.* (for example potatoes), *Chenopodiaceae sp.* (for example sugar beet, fodder beet, swiss chard, beetroot); useful plants and ornamental plants for gardens and wooded areas; and genetically modified varieties of each of these plants.

More specifically, the active substance is suitable for controlling viral diseases in vegetables. The active substance is particularly suitable for controlling viruses of the following families or genus: *Caulimoviridae, Geminiviridae, Bromoviridae, Closteroviridae, Comoviridae Potyviridae, Sequiviridae, Tombusviridae, Rhabdoviridae, Bunyaviridae, Partitiviridae, Rheoviridae, Capillovirus, Carlavirus, Enamovirus, Furovirus, Hordeivirus, Idaeovirus, Luteovirus, Marafivirus, Potexvirus, Sobemovirus, Tenuivirus, Tobamovirus, Tobravirus, Trichovirus, Tymovirus* and *Umbravirus.*

Preferably, the active substance is used for controlling viruses of the following species : Turnip mosaic virus, turnip crinkle virus, bean pod mottle virus, cauliflower mosaic virus, tobacco mosaic virus, tomato bushy stunt virus, rice ragged stunt virus, cucumber mosaic virus, barley yellow dwarf virus, beet yellows virus, lettuce yellows virus, maise mosaic virus, peanut stunt virus and potato virus Y.

The active substance can be applied to the plants in any suitable forms. For example, the active substance may be applied in the form of a suspension, e.g. water- or oil-based suspension, emulsion, solution, powder such as wettable powder, foam, paste, granules, microparticles, aerosols or microencapsulations. Suitable formulations can be prepared in conventional manners. The formulations comprising the active substance may be ready-for-use compositions, i.e. compositions that can be directly applied to the plants by a suitable device, or they may be in the form of commercial concentrates which have to be diluted prior to use.

The formulations may comprise the active substance alone or in combination with other active substances such as insecticides, attractants, sterilants, bactericides, acaricides, nematicides, further fungicides, growth-regulating substances, herbicides, safeners and/or fertilizers.

The active substance or formulations comprising thereof may be applied to the plants in any customary manners, e.g. watering, spraying, dusting, atomizing. The active substance may be directly or indirectly applied to the plants, the environment, the habitat and/or the store. For example, the active substance can be injected into or below the bark, poured or sprayed around the plant onto the ground (soil, sandy soil, gravelly soil, rocky soil, loamy soil or mixed soil). A further type of application is the spraying onto the plant and its plant parts. In dry form, the active substance composition can be admixed to the ground material (soil, sandy soil, gravelly soil, rocky soil, loamy soil or mixed soil) and/or to the seeds. The active substance can be applied to the irrigation system, either in dry or else in liquid form. The active substance is preferably applied to the plants by spraying.

The present invention also relates to the use of compounds of formula (I) as disclosed herein for controlling viral diseases in plants, more specifically for stimulating the natural defense mechanism of plants against viruses, in particular for stimulating the RNA-silencing-based defense mechanism of plants against viruses. The plants and/or viruses are as disclosed above.

The compounds of formula (I) stimulate the RNA-silencing-based defense mechanism of the plants through an increase in small RNA production. Advantageously, the ability of the compounds of formula (I) to increase small RNA production can also be used in genetically modified plants transformed with an RNAi construct designed to improve agronomic traits and/or to provide resistance against pathogens (e.g. bacteria, fungi), resistance against insect/pest and/or stress tolerance. Therefore, the present invention also relates to the use of one or more compounds of formula (I) to improve agronomic traits and/or to provide resistance against pathogens, resistance against insect/pest and/or stress tolerance in genetically modified plants transformed with an RNAi construct through enhancement of small RNA production.

The present invention also relates to a compound of formula (I) and/or a composition comprising thereof, wherein the compound of formula (I) is as follows: 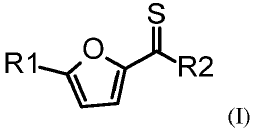 wherein
- R1 is a phenyl substituted with one or more halogens, preferably chlorine and bromine;
- R2 is a -NR'R" group wherein :
   - R' and R" are independently selected from the group consisting of (C₁-C₆)alkyl (preferably methyl, ethyl and n-propyl) and (C₃-C₆)cycloalkyl (preferably c-pentyl), or
   - R' and R" together with the nitrogen atom to which they are attached form a substituted piperidine or morpholine, wherein said substituted piperidine or morpholine is substituted with one or more substituents selected from the group consisting of halogen and (C₁-C₆)alkyl, or
   - R' and R" together with the nitrogen atom to which they are attached form an azabicycloalkyl that may be substituted with a (C₁-C₆)haloalkyl, preferably an azabicyclo[3.1.0]hexyl that may be substituted with a (C₁-C₆)haloalkyl.

In some embodiments, the compound is a compound of formula (I) wherein :
- R1 is a phenyl substituted with one or two halogens independently chosen from chlorine or bromine ; and/or
- R2 is a -NR'R" group wherein :
   - R' and R" are independently selected from the group consisting of methyl, ethyl, n-propyl and cyclopentyl, or
   - R' and R" together with the nitrogen atom to which they are attached form a substituted piperidine or morpholine, wherein said substituted piperidine or morpholine is substituted with one or more substituents selected from the group consisting of methyl and fluorine,
   - R' and R" together with the nitrogen atom to which they are attached form an azabicyclo[3.1.0]hexyl substituted with a (C₁-C₆)haloalkyl, such as a trifluoromethyl.

In some preferred embodiments, the compound is a compound of formula (I) wherein :
- R1 is 3,4-dichlorophenyl ; and/or
- R2 is selected from the group consisting of -NEt₂, -N(n-Pr)₂, -N(c-pentyl)Me, 4,4-difluoropiperidin-1-yl, 2,6-dimethylmorpholin-4-yl and 6-(trifluoromethyl)-3-azabicyclo[3.1.0]hex-3-yl.

The composition of the present invention typically comprises, in addition to the compound of formula (I), one or more acceptable carriers, in particular one or more agriculturally acceptable carriers.

A carrier is a natural or synthetic, organic or inorganic substance with which the active ingredients are mixed or combined for better applicability, in particular for better application to plants, plant parts or seeds. The carrier, which may be solid or liquid, is generally inert.

Examples of suitable solid carriers include, but are not limited to, ammonium salts, natural rock flours, such as kaolins, clays, talc, chalk, quartz, attapulgite, montmorillonite or diatomaceous earth, and synthetic rock flours, such as finely divided silica, alumina and silicates. Examples of typically useful solid carriers for preparing granules include, but are not limited to, crushed and fractionated natural rocks such as calcite, marble, pumice, sepiolite and dolomite, synthetic granules of inorganic and organic flours and granules of organic material such as paper, sawdust, coconut shells, maize cobs and tobacco stalks.

Examples of suitable liquid carriers include, but are not limited to, water, polar and nonpolar organic chemical liquids, for example from the classes of aromatic and nonaromatic hydrocarbons (such as cyclohexane, paraffins, alkylbenzenes, xylene, toluene alkylnaphthalenes, chlorinated aromatics or chlorinated aliphatic hydrocarbons such as chlorobenzenes, chloroethylenes or methylene chloride), alcohols and polyols (which may optionally also be substituted, etherified and/or esterified, such as butanol or glycol), ketones (such as acetone, methyl ethyl ketone, methyl isobutyl ketone cyclohexanone), esters (including fats and oils) and (poly)ethers, unsubstituted and substituted amines, amides (such as dimethylformamide),lactams (such as N-alkylpyrrolidones) and lactones, sulphones and sulphoxides (such as dimethyl sulphoxide). The carrier may also be a liquefied gaseous extender, i.e. liquid which is gaseous at standard temperature and under standard pressure, for example aerosol propellants such as halohydrocarbons, butane, propane, nitrogen and carbon dioxide.

The composition may further comprise one or more acceptable auxiliaries which are customary for formulating compositions (e.g. agrochemical compositions), such as one or more surfactants. Examples of suitable surfactants include emulsifiers and/or foam formers, dispersants or wetting agents having ionic or nonionic properties, or mixtures thereof. Examples thereof are salts of polyacrylic acid, salts of lignosulphonic acid, salts of phenolsulphonic acid or naphthalenesulphonic acid, polycondensates of ethylene and/or propylene oxide with fatty alcohols, fatty acids or fatty amines (polyoxyethylene fatty acid esters, polyoxyethylene fatty alcohol ethers, for example alkylaryl polyglycol ethers), substituted phenols (preferably alkylphenols or arylphenols), salts of sulphosuccinic esters, taurine derivatives (preferably alkyl taurates), phosphoric esters of polyethoxylated alcohols or phenols, fatty esters of polyols, and derivatives of compounds containing sulphates, sulphonates and phosphates, for example alkylaryl polyglycol ethers, alkylsulphonates, alkyl sulphates, arylsulphonates, protein hydrolysates, lignosulphite waste liquors and methylcellulose. A surfactant is typically used when the active ingredient and/or the carrier is insoluble in water and the application is made with water. Then, the amount of surfactants typically ranges from 5 to 40 % by weight of the composition.

Further examples of auxiliaries which are customary for formulating agrochemical compositions include water repellent, siccatives, binder (adhesive, tackifier, fixing agent, such as carboxymethylcellulose, natural and synthetic polymers in the form of powders, granules or latices, such as gum arabic, polyvinyl alcohol and polyvinyl acetate, natural phospholipids such as cephalins and lecithins and synthetic phospholipids, polyvinylpyrrolidone, polyvinyl acetate, polyvinyl alcohol and tylose), thickeners, stabilizers (e.g. cold stabilizers, preservatives, antioxidants, light stabilizers, or other agents which improve chemical and/or physical stability), dyes or pigments (such as inorganic pigments, e.g. iron oxide, titanium oxide and Prussian Blue ; organic dyes, e.g. alizarin, azo and metal phthalocyanine dyes), antifoams (e.g. silicone antifoams and magnesium stearate), preservatives (e.g. dichlorophene and benzyl alcohol hemiformal), secondary thickeners (cellulose derivatives, acrylic acid derivatives, xanthan, modified clays and finely divided silica), stickers, gibberellins and processing auxiliaries, mineral and vegetable oils, perfumes, waxes and nutrients (including trace nutrients, such as salts of iron, manganese, boron, copper, cobalt, molybdenum and zinc), protective colloids, thixotropic substances, penetrants, sequestering agents and complex formers. The choice of the carriers and/or auxiliaries will depend on the intended mode of application of the composition and/or on the physical properties of the active ingredient(s).

The present invention is explained in greater detail with the aid of the examples which follow.

### EXAMPLES

### Example 1 : Identification of modulators of plant's silencing machinery - use of SUC-SUL reporter plants

The plants' RNA silencing pathway can be easily monitored with certain artificially-created reporter plants called the "SUC-SUL *Arabidopsis* reporter plants" (Dunoyer et al., Nat. Genet. 37, 1356-1360, 2005).

The SUC-SUL *Arabidopsis* reporter plants are transgenic plants expressing an inverted-repeat (IR) double-stranded RNA designed to target the SULPHUR (SUL) transcript in the vasculature under the control of the SUC2 promoter (Truernit et al., Planta 196(3), 564-570, 1995). Once expressed, the dsRNA is processed into small interfering RNAs directing non-cell autonomous post-transcriptional gene silencing of the SULPHUR transcript, in turn causing vein-centered chlorosis. Since the observed chlorosis results from the silencing of the SULPHUR transcript, an expansion of the chlorosis, observable directly on the plants, is correlated with an intensification of the RNA silencing pathway at the molecular level.

Five days post-germination A. *thaliana* seedlings (n=6) were sprayed with different solutions containing 300 ppm of an active molecule according to the invention, with dimethylsulfoxide (DMSO) 5 % and a standard emulsifiable concentrate (EC) premix formulation (6 seedlings/test). Four repetitions were performed for each molecule. Control plants were treated with DMSO only (mock treated plants). The effects of the treatments on the SUC-SUL reporter plants were assessed 14 days after treatment under trans-illumination and digitally recorded with a stereo-microscope by measuring the surface and intensity of the chlorotic zone (percentage of chlorosis). Two independent experiments were carried out in order to estimate the average percentage of chlorosis for each molecule.

Results are shown in table 1 below. Treatment with 300 ppm of DFPM ([5-(3,4-dichlorophenyl)furan-2-yl]-piperidine-1-ylmethanethione) and analogues thereof led to a significant increase in the percentage of chlorosis confirming the activity of the active substance as putative robust enhancers of RNA silencing.

In order to discard molecules acting straightly on the promoter and not on the RNA silencing machinery, the active substances identified for their ability to increase the surface and intensity of the chlorotic zone were then tested, in a second step, on some AtSUC2-GFP reporter plants, which are specifically reporting the activity of the SUC2 promoter (Wright et al., Plant Physiol. 131, 1555-1565, 2003).

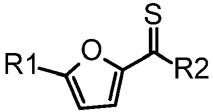

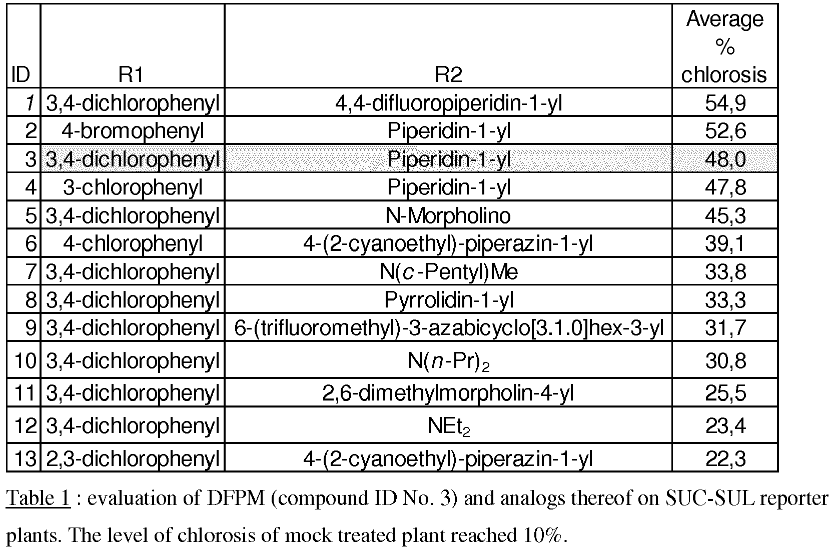

### Example 2 : Confirmation of the molecular effect of the silencing modulators

The enhancing effect of DFPM on the RNAi machinery has been validated at the phenotypic and molecular levels. Four-week-old SUC-SUL *Arabidopsis* plants at rosette stage were sprayed with DFPM at 200 ppm. Seven days after treatment, it was shown that DFMP induced a clear expansion of the RNAi-dependent vein-centered chlorotic phenotype, which was not observed with DMSO only (mock control).

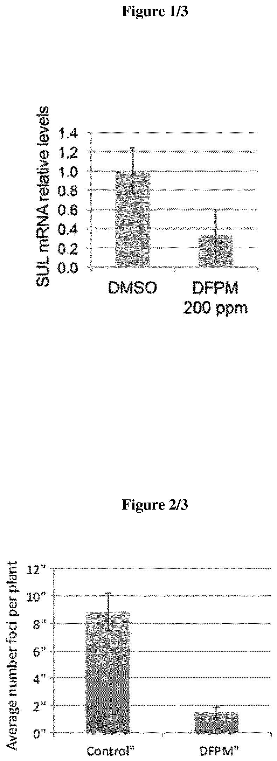

The plant aerial tissues were collected and analyzed molecularly using state-of-the-art methodologies. The visual expansion of the chlorosis correlated with reductions in *SUL* transcript levels, as analyzed by real-time qRT-PCR (Figure 1), as well as reductions in SUL protein levels quantifiable on Western blot analyses. Furthermore, these effects were associated with an over-accumulation of both SUL 21-nt and 24-nt long siRNAs derived from the transgenic *SUL.* Altogether, these data support that DFPM induces the plant's RNAi pathway.

### Example 3 : Antiviral efficacy of silencing modulators on Arabidopsis

The Turnip mosaic virus (TuMV) belongs to the *Potyviridae,* the largest family of known plant viruses. This virus infects a wide range of plants, in particular *Brassicaceae.* TuMV genome consists of a positive-sense single-stranded RNA molecule (+ ssRNA). The antiviral efficacy of DFMP was tested with a fluorescently-tagged potyvirus (TuMV-GFP) in order to specifically pinpoint when and where the effect of the molecules is induced during virus primary infection, as well as its local or systemic spread.

*Arabidopsis* plants were sprayed with 100 or 200 ppm of DFMP 3 days before infecting the plants with the TuMV-GFP virus. Virus titers were measured by qPCR and/or virus protein accumulation on Western blot analyses and by counting the number of primary infection foci under UV light in the case of the TuMV-GFP.

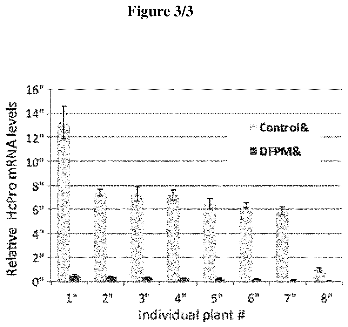

DFPM leads to a clear reduction of the accumulation of virus titers five days-post-infection on the *Arabidopsis* plants. A strong reduction of virus accumulation was consistently observed with TuMV-GFP both on inoculated leaves (Figure 2) and on systemic tissues upon treatment with DFPM, as measured by the reduction of the accumulation of the TuMV-derived HcPro mRNA levels (Figure 3).

Similar results were obtained with DFPM with the unrelated viruses *Turnip crinkle virus* (TCV,) and *Cauliflower mosaic virus* (CaMV). The TCV belongs to the *Tombusviridae* family and infects various types of plant species including the common plant models *A*. *thaliana* and *N. benthamiana.* Its genome is another example of +strand RNA virus. The CaMV belongs to the *Caulimoviridae* family. It infects mostly *Brassicaceae* such as turnip and cabbage, but is also able to infect A. *thaliana.* CaMV consists of a double-stranded DNA molecule.

DFPM treatment at 100ppm lead to a strong reduction of virus accumulation both on inoculated leaves and on systemic tissues, as measured by the reduction of the accumulation of the TCV-derived P38 mRNA and CaMV-derived P6 mRNA.

### Example 4 : Antiviral efficacy of silencing modulators on Soybean plants

The Bean pod mottle virus (BPMV) is a plant pathogenic virus of the family Comoviridae. This virus infects legumes and more particularly soybean. BPMV genome consists of a bipartite positive-sense single-stranded RNA molecule (+ ssRNA). The antiviral efficacy of silencing enhancers was tested with a fluorescently-tagged comoviruss (BPMV-GFP) in order to specifically pinpoint when and where the effect of the molecules is induced during virus primary infection, local or systemic spread.

Soybean plants were sprayed with 100 or 200ppm of DFPM and emulsifiable concentrate (EC) Premix one day before to infect the plants with the BPMV-GFP virus. Twelve replicates were used for each condition. DFPM lead to a clear reduction of the number of plants showing BPMV symptoms and a clear reduction of the BPMV fluorescence on soybean seven days-post-infection on soybean (Table 2).

**Table 2: Reduction of BMPV symptoms and fluorescence upon treatment with DFPM at 100 and 200 ppm.**

| Treatment | Percentage of plants showing BPMV symptoms | Average of Percentage of BPMV fluorescence |
|---|---|---|
| water | 83% | 46% |
| EC formulation | 63% | 31% |
| DFPM 100ppm | 55% | 31% |
| DFPM 200ppm | 10% | 6% |

## Claims

1. A method for controlling viral diseases in plants, which comprises applying to the plants at least one compound of formula (I) : 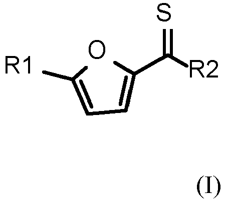 wherein
- R1 is a phenyl that may be substituted with one or more halogens ;
- R2 is a -NR'R" group wherein :
- R' and R" are independently selected from the group consisting of (C₁-C₆)alkyl and (C₃-C₆)cycloalkyl, or
- R' and R" together with the nitrogen atom to which they are attached form a 5- or 6-membered heterocycle chosen from pyrrolidine, piperidine, morpholine, or piperazine, wherein said heterocycle may be substituted with one or more substituents selected from the group consisting of halogen, (C₁-C₆)alkyl and (C₁-C₆)cyanoalkyl, or
- R' and R" together with the nitrogen atom to which they are attached form an azabicycloalkyl that may be substituted with a (C₁-C₆)haloalkyl.

2. The method according to claim 1, wherein R1 is selected from the group consisting of 3-chlorophenyl, 4-chlorophenyl, 4-bromophenyl, 2,3-dichlorophenyl and 3,4-dichlorophenyl.

3. The method according to claim 1 or 2 wherein R2 is selected from the group consisting of - NEt₂, -N(n-Pr)₂, -N(c-pentyl)Me, piperidin-1-yl, pyrrolidin-1-yl, N-morpholino, 4,4-difluoropiperidin-1-yl, 4-(2-cyanoethyl)-piperazin-1-yl, 2,6-dimethylmorpholin-4-yl and 6-(trifluoromethyl)-3-azabicyclo[3.1.0]hex-3-yl.

4. The method according to anyone of claims 1 to 3, wherein the control is based on a stimulation of the natural defense mechanism of plants against viruses.

5. The method according to claim 4, wherein the natural defense mechanism of plants against viruses is a RNA-silencing based plant defense mechanism.

6. The method according to anyone of the preceding claims, wherein said method is a preventive method.

7. The method according to anyone of the preceding claims, wherein said plants are selected from the group consisting of cotton, flax, grapevine, fruit, vegetables, crop plants, ornamental plants for gardens and wooded area and genetically modified varieties of each of these plants.

8. The method according to anyone of the preceding claims, wherein said plants are selected from the group consisting of *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp., Rubiaceae sp., Theaceae sp., Sterculiceae sp., Rutaceae sp.; Solanaceae sp., Liliaceae sp., Asteraceae sp., Umbelliferae sp., Cruciferae sp., Chenopodiaceae sp., Cucurbitaceae sp., Alliaceae sp., Papilionaceae sp., Asteraceae sp., Brassicaceae sp., Fabacae sp., Papilionaceae sp., Solanaceae sp.* and *Chenopodiaceae sp..*

9. The method according to anyone of the preceding claims, wherein said viruses are selected from the group consisting of the following families or genus: *Caulimoviridae, Geminiviridae, Bromoviridae, Closteroviridae, Comoviridae Potyviridae, Sequiviridae,* Tombusviridae, *Rhabdoviridae, Bunyaviridae, Partitiviridae, Rheoviridae, Capillovirus, Carlavirus, Enamovirus, Furovirus, Hordeivirus, Idaeovirus, Luteovirus, Marafivirus, Potexvirus, Sobemovirus, Tenuivirus, Tobamovirus, Tobravirus, Trichovirus, Tymovirus* and *Umbravirus.*

10. The method according to anyone of the preceding claims, wherein said viruses are selected from the group consisting of Turnip mosaic virus, turnip crinkle virus, bean pod mottle virus, cauliflower mosaic virus, tobacco mosaic virus, tomato bushy stunt virus, rice ragged stunt virus, cucumber mosaic virus, barley yellow dwarf virus, beet yellows virus, lettuce yellows virus, maise mosaic virus, peanut stunt virus and potato virus Y.

11. The method according to anyone of the preceding claims, wherein the compound of formula (I) is applied by spraying to the plants.

12. The method according to anyone of the preceding claims, wherein the compound of formula (I) is applied to the plants in an amount ranging from 0.01 to 5kg/ha.

13. Use of a compound of formula (I) as defined in claim 1, 2 or 3 for stimulating the natural defense mechanism of plants against viruses.

14. Use according to claim 13 for stimulating the RNA-silencing-based plant defense mechanism.

15. Use of a compound of formula (I) as defined in claim 1, 2 or 3 to improve agronomic traits and/or to provide resistance against pathogens, insect, pest and/or to provide stress tolerance in genetically modified plants transformed with an RNAi construct.

16. A compound of formula (I) : 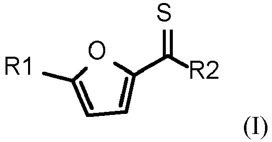 wherein
- R1 is a phenyl substituted with one or more halogens ;
- R2 is a -NR'R" group wherein :
- R' and R" are independently selected from the group consisting of (C₁-C₆)alkyl and (C₃-C₆)cycloalkyl, or
- R' and R" together with the nitrogen atom to which they are attached form a substituted piperidine or morpholine, wherein said substituted piperidine or morpholine is substituted with one or more substituents selected from the group consisting of halogen and (C₁-C₆)alkyl, or
- R' and R" together with the nitrogen atom to which they are attached form an azabicycloalkyl that may be substituted with a (C₁-C₆)haloalkyl.

## Patentansprüche

1. Verfahren zur Bekämpfung von Viruskrankheiten in Pflanzen, welches das Ausbringen mindestens einer Verbindung der Formel (I): 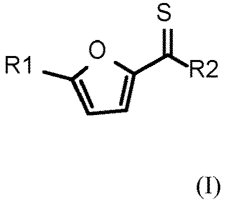 auf die Pflanzen umfasst, wobei
- R1 für Phenyl steht, dass durch ein oder mehrere Halogene substituiert sein kann,
- R2 für eine -NR'R"-Gruppe steht, wobei:
- R' und R" unabhängig voneinander aus der aus (C₁-C₆)-Alkyl und (C₃-C₆)-Cycloalkyl bestehenden Gruppe ausgewählt sind oder
- R' und R" zusammen mit dem Stickstoffatom, an das sie gebunden sind, einen aus Pyrrolidin, Piperidin, Morpholin und Piperazin ausgewählten 5- oder 6-gliedrigen Heterocyclus bilden, wobei der Heterocyclus durch einen oder mehrere aus der aus Halogen, (C₁-C₆)-Alkyl und (C₁-C₆)-Cyanoalkyl bestehenden Gruppe ausgewählte Substituenten substituiert sein kann, oder
- R' und R" zusammen mit dem Stickstoffatom, an das sie gebunden sind, einen Azabicycloalkylrest bilden, der durch (C₁-C₆)-Halogenalkyl substituiert sein kann.

2. Verfahren nach Anspruch 1, wobei R1 aus der aus 3-Chlorphenyl, 4-Chlorphenyl, 4-Bromphenyl, 2,3-Dichlorphenyl und 3,4-Dichlorphenyl bestehenden Gruppe ausgewählt ist.

3. Verfahren nach Anspruch 1 oder 2, wobei R2 aus der aus NEt₂, -N(n-Pr)₂, -N(c-Pentyl)Me, Piperidin-1-yl, Pyrrolidin-1-yl, N-Morpholino, 4,4-Difluorpiperidin-1-yl, 4-(2-Cyanoethyl)piperazin-1-yl, 2,6-Dimethylmorpholin-4-yl und 6-(Trifluormethyl)-3-azabicyclo[3.1.0]hex-3-yl bestehenden Gruppe ausgewählt ist.

4. Verfahren nach einem der Ansprüche 1 bis 3, wobei die Bekämpfung auf einer Stimulierung der natürlichen Abwehrmechanismen von Pflanzen gegen Viren basiert.

5. Verfahren nach Anspruch 4, wobei es sich bei dem natürlichen Abwehrmechanismus von Pflanzen gegen Viren um einen auf RNA-Silencing basierenden Pflanzenabwehrmechanismus handelt.

6. Verfahren nach einem der vorhergehenden Ansprüche, wobei es sich bei dem Verfahren um ein präventives Verfahren handelt.

7. Verfahren nach einem der vorhergehenden Ansprüche, wobei die Pflanzen aus der aus Baumwolle, Flachs, Weinrebe, Früchten, Gemüsen, Kulturpflanzen, Zierpflanzen für Gärten und bewaldete Flächen und genetisch modifizierten Sorten jeder dieser Pflanzen bestehenden Gruppe ausgewählt sind.

8. Verfahren nach einem der vorhergehenden Ansprüche, wobei die Pflanzen aus der aus *Ribesioidae sp.* , *Juglandacea sp., Betulacea sp., Anacardiaceae sp., Fagaceae sp., Moraceae sp., Oleaceae sp., Actinidaceae sp., Lauraceae sp., Musaceae sp., Rubiaceae sp., Theaceae sp., Sterculiceae sp., Rutaceae sp.*, *Solanaceae sp.*, *Liliaceae sp.*, *Asteraceae sp., Umbelliferae sp., Cruciferae sp., Chenopodiaceae sp., Cucurbitaceae sp., Alliaceae sp., Papilionaceae sp., Asteraceae sp., Brassicaceae sp., Fabacae sp., Papilionaceae sp., Solanaceae sp.* und *Chenopidiaceae sp.* bestehenden Gruppe ausgewählt sind.

9. Verfahren nach einem der vorhergehenden Ansprüche, wobei die Viren aus der aus den folgenden Familien bzw. Gattungen bestehenden Gruppe ausgewählt sind: *Caulimoviridae, Geminiviridae, Bromoviridae, Closteroviridae, Comoviridae, Potyviridae, Sequiviridae,* Tombusviridae, *Rhabdoviridae, Bunyaviridae, Partitiviridae, Rheoviridae, Capillovirus, Carlavirus, Enamovirus, Furovirus, Hordeivirus, Idaeovirus, Luteovirus, Marafivirus, Potexvirus, Sobemovirus, Tenuivirus, Tobamovirus, Tobravirus, Trichovirus, Tymovirus* und *Umbravirus.*

10. Verfahren nach einem der vorhergehenden Ansprüche, wobei die Viren aus der aus Rübenmosaikvirus, Kräuselkrankheit der Rübe (Turnip Crinkle Virus), Bohnenhülsenscheckungsvirus (Bean Pod Mottle Virus), Blumenkohlmosaikvirus, Tabakmosaikvirus, Tomato Bushy Stunt Virus, Rice Ragged Stunt Virus, Gurkenmosaikvirus, Gelbverzwergungsvirus (Barley Yellow Dwarf Virus), Rübenvergilbungsvirus, Salatvergilbungsvirus, Maismosaikvirus, Peanut Stunt Virus und Kartoffelvirus Y bestehenden Gruppe ausgewählt ist.

11. Verfahren nach einem der vorhergehenden Ansprüche, wobei die Verbindung der Formel (I) durch Sprühen auf die Pflanzen aufgebracht wird.

12. Verfahren nach einem der vorhergehenden Ansprüche, wobei die Verbindung der Formel (I) auf die Pflanzen in einer Menge im Bereich von 0,01 bis 5 kg/ha aufgebracht wird.

13. Verwendung einer wie in Anspruch 1, 2 oder 3 definierten Verbindung der Formel (I) zum Stimulieren der natürlichen Abwehrmechanismen von Pflanzen gegen Viren.

14. Verwendung nach Anspruch 13 zum Stimulieren des auf RNA-Silencing basierenden Pflanzenabwehrmechanismus.

15. Verwendung einer wie in Anspruch 1, 2 oder 3 definierten Verbindung der Formel (I) zum Verbessern agronomischer Merkmale und/oder zur Bereitstellung von Resistenz gegen Pathogene, Insekten, Schädlinge und/oder zur Bereitstellung von Stresstoleranz in mit einem RNAi-Konstrukt transformierten genetisch modifizierten Pflanzen.

16. Verbindung der Formel (I): 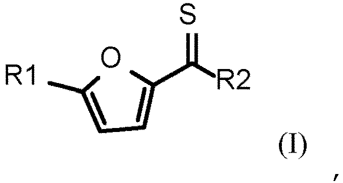 wobei
- R1 für Phenyl steht, das durch ein oder mehrere Halogene substituiert sein kann,
- R2 für eine -NR'R"-Gruppe steht, wobei:
- R' und R" unabhängig voneinander aus der aus (C₁-C₆)-Alkyl und (C₃-C₆)-Cycloalkyl bestehenden Gruppe ausgewählt sind oder
- R' und R" zusammen mit dem Stickstoffatom, an das sie gebunden sind, ein substituiertes Piperidin oder Morpholin bilden, wobei das substituierte Piperidin bzw. Morpholin durch einen oder mehrere aus der aus Halogen und (C₁-C₆)-Alkyl bestehenden Gruppe ausgewählte Substituenten substituiert ist, oder
- R' und R" zusammen mit dem Stickstoffatom, an das sie gebunden sind, einen Azabicycloalkylrest bilden, der durch (C₁-C₆) - Halogenalkyl substituiert sein kann.

## Revendications

1. Méthode de contrôle de maladies virales chez les plantes, comprenant l'application aux plantes d'au moins un composé de formule (I) 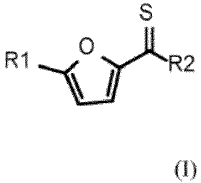 dans laquelle
- R1 est phényle pouvant être substitué par un ou plusieurs halogènes ;
- R2 est un groupement -NR'R" où
- R' et R" sont choisis indépendamment dans le groupe constitué par (C₁-C₆)alkyle et (C₃-C₆)cycloalkyle, ou
- R' et R", conjointement avec l'atome d'azote auquel ils sont fixés, forment un hétérocycle de 5 ou 6 chaînons choisi parmi pyrrolidine, pipéridine, morpholine, ou pipérazine, où ledit hétérocycle peut être substitué par un ou plusieurs substituants choisis dans le groupe constitué par halogène, (C₁-C₆)alkyle et (C₁-C₆)cyanoalkyle, ou
- R' et R", conjointement avec l'atome d'azote auquel ils sont fixés, forment un azabicycloalkyle pouvant être substitué par (C₁-C₆)halogénoalkyle.

2. Méthode selon la revendication 1, dans laquelle R1 est choisi dans le groupe constitué par 3-chlorophényle, 4-chlorophényle, 4-bromophényle, 2,3-dichlorophényle et 3,4-dichlorophényle.

3. Méthode selon la revendication 1 ou 2, dans laquelle R2 est choisi dans le groupe constitué par -NEt₂, -N(n-Pr)₂, -N(c-pentyl)Me, pipéridin-1-yle, pyrrolidin-1-yle, N-morpholino, 4,4-difluoropipéridin-1-yle, 4-(2-cyanoéthyl)pipérazin-1-yle, 2,6-diméthylmorpholin-4-yle et 6-(trifluorométhyl)-3-azabicyclo[3.1.0]hex-3-yle.

4. Méthode selon l'une quelconque des revendications 1 à 3, dans laquelle le contrôle est basé sur une stimulation du mécanisme de défense naturel des plantes contre les virus.

5. Méthode selon la revendication 4, dans laquelle le mécanisme de défense naturel des plantes contre les virus est un mécanisme de défense des plantes basé sur le silençage d'ARN.

6. Méthode selon l'une quelconque des revendications précédentes, **caractérisée en ce que** ladite méthode est une méthode préventive.

7. Méthode selon l'une quelconque des revendications précédentes, dans laquelle lesdites plantes sont choisies dans le groupe constitué par le coton, le lin, la vigne, les fruits, les légumes, les plantes de culture, les plantes ornementales pour les jardins et les zones boisées ainsi que les variétés génétiquement modifiées de chacune de ces plantes.

8. Méthode selon l'une quelconque des revendications précédentes, dans laquelle lesdites plantes sont choisies dans le groupe constitué par *Ribesioidae* sp., *Juglandaceae sp., Betulaceae sp., Anacardiaceae sp., Fagaceae sp., Moraceae sp., Oleaceae sp., Actinidaceae sp., Lauraceae sp., Musaceae sp., Rubiaceae sp., Theaceae sp., Sterculiceae sp., Rutaceae sp., Solanaceae sp.*, *Liliaceae sp., Asteraceae sp., Umbelliferae* sp., *Cruciferae sp., Chenopodiaceae sp., Cucurbitaceae sp.*, *Alliaceae sp., Papilionaceae sp., Asteraceae sp., Brassicaceae sp., Fabacae sp., Papilionaceae sp., Solanaceae sp.* et *Chenopodiaceae sp..*

9. Méthode selon l'une quelconque des revendications précédentes, dans laquelle lesdits virus sont choisis dans le groupe constitué par les familles ou genres suivant(e)s : *Caulimoviridae, Geminiviridae, Bromoviridae, Closteroviridae, Comoviridae, Potyviridae, Sequiviridae, Tombusviridae, Rhabdoviridae, Bunyaviridae, Partitiviridae, Reoviridae, Capillovirus, Carlavirus, Enamovirus, Furovirus, Hordeivirus, Idaeovirus, Luteovirus, Marafivirus, Potexvirus, Sobemovirus, Tenuivirus, Tobamovirus, Tobravirus, Trichovirus,* Tymovirus et *Umbravirus.*

10. Méthode selon l'une quelconque des revendications précédentes, dans laquelle lesdits virus sont choisis dans le groupe constitué par le virus de la mosaïque du navet, le virus du rabougrissement du navet, le virus de la marbrure des gousses de haricot, le virus de la mosaïque du chou-fleur, le virus de la mosaïque du tabac, le virus du rabougrissement buissonneux de la tomate, le virus du rabougrissement rugueux du riz, le virus de la mosaïque du concombre, le virus de la jaunisse nanisante de l'orge, le virus de la jaunisse de la betterave, le virus de la jaunisse de la laitue, le virus de la mosaïque du maïs, le virus du rabougrissement de l'arachide et le virus Y de la pomme de terre.

11. Méthode selon l'une quelconque des revendications précédentes, dans laquelle le composé de formule (I) est appliqué par pulvérisation aux plantes.

12. Méthode selon l'une quelconque des revendications précédentes, dans laquelle le composé de formule (I) est appliqué aux plantes selon une quantité allant de 0,01 à 5 kg/ha.

13. Utilisation d'un composé de formule (I) tel que défini selon la revendication 1, 2 ou 3, pour la stimulation du mécanisme de défense naturel des plantes contre les virus.

14. Utilisation selon la revendication 13, pour la stimulation du mécanisme de défense des plantes basé sur le silençage d'ARN.

15. Utilisation d'un composé de formule (I) tel que défini selon la revendication 1, 2 ou 3, pour l'amélioration des caractères agronomiques et/ou pour l'apport d'une résistante contre des pathogènes, des insectes, des nuisibles et/ou pour l'apport d'une tolérance au stress chez des plantes génétiquement modifiées transformées par un produit de construction d'ARNi.

16. Composé de formule (I) 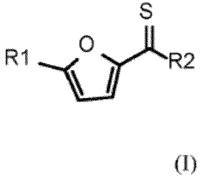 dans lequel
- R1 est phényle substitué par un ou plusieurs halogènes ;
- R2 est un groupement -NR'R" où
- R' et R" sont choisis indépendamment dans le groupe constitué par (C₁-C₆)alkyle et (C₃-C₆)cycloalkyle, ou
- R' et R", conjointement avec l'atome d'azote auquel ils sont fixés, forment une pipéridine ou morpholine substituée, où ladite pipéridine ou morpholine substituée est substituée par un ou plusieurs substituants choisis dans le groupe constitué par halogène et (C₁-C₆)alkyle, ou
- R' et R", conjointement avec l'atome d'azote auquel ils sont fixés, forment un azabicycloalkyle pouvant être substitué par (C₁-C₆)halogénoalkyle.
